# EUROPÄISCHE PATENTSCHRIFT

(11) **EP 2 344 252 B1**
(45) Veröffentlichungstag und Bekanntmachung des Hinweises auf die Patenterteilung: **12.07.2017**
(21) Anmeldenummer: 09748986.8
(22) Anmeldetag: 12.10.2009
(51) Int. Cl.: A61N 7/00, A61B 17/32

(54) **VORRICHTUNG ZUM EINLEITEN VON STOSSWELLEN IN EINEN LEBENDEN KÖRPER UND DEREN VERWENDUNG**
DEVICE FOR INTRODUCING SHOCK WAVES INTO A LIVING BODY AND USE THEREOF
DISPOSITIF POUR ENVOYER DES ONDES DE CHOC DANS UN CORPS VIVANT ET SON UTILISATION

(30) Priorität: 14.10.2008 DE 102008051174; 22.09.2009 DE 102009042276
(43) Veröffentlichungstag der Anmeldung: 20.07.2011
(73) Patentinhaber: Ferton Holding SA, 2800 Delemont (CH)
(72) Erfinder: FÖHRENBACH, Marianne, 78159 Döggingen (DE)
(74) Vertreter: Patentanwälte und Rechtsanwalt Weiß, Arat & Partner mbB
(86) Internationale Anmeldenummer: PCT/EP2009/007322
(87) Internationale Veröffentlichungsnummer: WO 2010/043360

(56) Entgegenhaltungen:
- DE-A1- 3 315 185
- DE-U1-202006 007 044
- FR-A1- 2 851 153
- US-A- 1 599 628
- US-A- 1 657 765
- US-A- 1 796 444
- US-A- 4 088 128
- US-A- 5 160 336

## Beschreibung

Die Erfindung betrifft ein Verfahren und eine Vorrichtung zum Einleiten von Stosswellen in einen lebenden Körper entsprechend dem Oberbegriff von Anspruch 1.

Die Vorrichtung ist vorzugsweise als medizinisches Handgerät konzipiert, das sowohl bei der intrakorporalen Stosswellen-Lithotripie als auch bei der extrakorporalen Stosswellen-Orthopädie im Rahmen von der Zertrümmerung von Körpersteinen oder oberflächlich bei erkrankten Körperpartien verwendet werden kann.

### Stand der Technik

Vibration- bzw. Massageräte sind in vielfältiger Form und Ausführung bekannt und auf dem Markt. So zeigt beispielsweise die US 1 796 444 einen Vibrator, bei dem ein gummiartiger Massagekopf über eine Stange mit einer Einrichtung verbunden ist, welche die Stange gegen die Kraft einer Schraubenfeder anhebt, wobei die Stange unter der Kraft der Schraubenfeder wieder abgesenkt wird. Durch diese reziprozierende Bewegung des Massagekopfes kann eine Vibration auch den Bereich von zum Beispiel einem menschlichen Körper übertragen werden oder auch eine Massage stattfinden. Ein Zertrümmern von Konkrementen im Körper kann aber nicht durchgeführt werden, da keine Stosswelle durch die reziprozierende Bewegung erzeugt werden. Ähnlich Massagegeräte sind auch aus der DE 33 15 185 A1, der US 1 599 628 oder US 4 088 128 bekannt.

Ein weiteres Massagerät betrifft die US 1 657 765, wobei dort der Massagekopf direkt über eine Spanneinrichtung mit dem Federglied verbunden ist. Die Spanneinrichtung wird mittels einer exzentrischen Kulisse angehoben und dann ausgelöst, so dass der Massagekopf unter dem Druck des Federgliedes schlagartig auf den Körper auftrifft. Die Kulisse selbst wird durch eine Wirkverbindung zwischen einem Zahnkranz und einer Schnecke bewegt. Diese Anordnung hat allerdings den Nachteil, dass auch hier der Massagekopf selbst die reziprozierende Bewegung des Schlagteils mitmacht, so dass er dauernd gegenüber den zu behandelnden Bereich bewegt wird und keine Stosswellen ausgehend von dem Massagekopf erzeugt werden.

Die Zertrümmerung von Konkrementen in viva durch den Einsatz von Stosswellen (Lithotripsie oder Lithotropie) wird in der modernen Medizin bereits mit Erfolg angewendet. Die Konkremente, wie z.B. Nierensteine, werden dabei im Körper eines Menschen zu kleinen Fragmenten zertrümmert, die dann diesen über die Harnwege verlassen sollen. Im vorliegenden Fall wird unter Stosswelle insbesondere jede durch einen Aufschlag erzeugte Kompressionswelle verstanden.

Der Stand der Technik beschreibt verschiedene Vorrichtungen und Verfahren für die Erzeugung von Stosswellen zum Zertrümmern von Konkrementen in einem lebenden Körper. So beschreibt z.B. das US-Patent Nr. 5,868,756 ein Handgerät zur Verwendung bei der intrakorporalen Stosswellen-Lithotripsie, welches eine Sonde aufweist, die als ein Wellenleiter zur Übertragung von Stosswellen dient und zur Einführung in ein Endoskop entsprechend bemessen ist.

Die Stossenergie für die Erzeugung der Stosswellen wird bei diesem Handgerät durch ein in einem Führungsrohr pneumatisch hin- und her bewegbares Schlagteil oder Projektil erhalten, und zwar indem dieses periodisch auf ein entsprechend grosses Masseteil schlägt, welches dann die auf deren Trefffläche einwirkende Stosskraft an die an das Masseteil angrenzende Sonde weiterleitet.

In dieser US-Schrift ist auch angegeben, dass anstelle eines pneumatischen Antriebs für das Schlagteil auch alternativ ein hydraulischer oder elektromagnetischer Antrieb realisiert werden kann.

Ähnliche mit Druckluft arbeitende Stosswellengeneratoren sind aus der US 5 160 336, der DE 20 2006 008 044 U1 und der FR 2 852 153 bekannt.

Ferner ist in der DE 197 25 477 C2 zur Behandlung von biologischem Gewebe (Orthopädie) ein medizinisches Instrument in der Form eines Handgerätes beschrieben, durch welches extrakorporale Druckwellen erzeugt werden sollen. Diese Druckwellen sollen dabei mit Hilfe eines Übertragungselements in den Körper von Lebewesen gebracht werden. Hierfür besitzt das Übertragungselement eine stumpfe Sondenspitze mit einer flachen Austrittsgrenzfläche, mit der dann unfokussierte, mechanisch erzeugte Druckwellen in das biologische Gewebe eingekoppelt werden. Die Druckwelle selbst wird bei diesem Instrument von einem auf eine Geschwindigkeit von über 5 m/s pneumatisch beschleunigten und auf das Übertragungselement auftreffenden Schlagteil erzeugt, wobei dabei die Sondenspitze einen Hub von weniger als 1 mm ausführen soll. Mit diesem Handgerät soll ferner eine gleichmässige Energieverteilung der Druckwellen auf einen grossflächigen Wirkungsbereich erreicht werden, wie z.B. ein gesamter Entzündungsbereich.

Die bekannten Handgeräte besitzen jedoch alle Druck- bzw. Stosswellengeneratoren, sind relativ aufwendig konzipiert, da Kompressor, Regeleinheit und Handstück benötigt werden.

Eine Vorrichtung der eingangs erwähnten Art ist beispielweise aus der US 4, 549, 535 allgemein zur Behandlung von Menschen bekannt. Da sie eine weiche Spitze besitzt, ist sie nicht für die Lithotropsie einsetzbar.

### Aufgabe der Erfindung

Es ist daher die Aufgabe der vorliegenden Erfindung, eine Vorrichtung zum Einleiten oder Aufbringen von Stosswellen in oder auf einen lebenden Körper zu schaffen, dessen Stosswellengenerator zum einen einfach aufgebaut ist und zum anderen eine exakte "harte" Schlagkraft liefert. Ferner soll die Vorrichtung wartungsarm sein und sich sowohl für eine intra- als auch für eine extrakorporale Behandlung, vorzugsweise am menschlichen Körper, gut eignen.

### Lösung der Aufgabe

Diese Aufgabe wird erfindungsgemäss durch die kennzeichnenden Merkmale von Anspruch 1 gelöst.

Durch einen derart aufgebauten Stosswellengenerator ist es vorteilhaft möglich, dass die Stosswellen übertragende Sonde mit relativ hoher ("harter") Schlagenergie periodisch beaufschlagt werden kann, und zwar ähnlich wie die Schussfolge bei einem Maschinengewehr, weswegen der vorliegende Erfindungsgegenstand auch als Lithogun bzw. Orthogun bezeichnet werden kann. Die "harte" Schlagkraft wird vor allem durch ein massearmes Element mit hoher Aufschlaggeschwindigkeit erzielt.

Nach Massgabe der vorliegenden Erfindung ist es zweckmässig, dass als Federglied bevorzugt eine pneumatische oder zylindrische mechanische Druckfeder dient, die mit einem Schlagteil derart verbunden ist, dass dieses die Federkraft der Druckfeder bei einer plötzlichen Freigabe ihrer Druckspannung in der Form eine Stossimpulses auf das übertragende Element abgibt.

Hierbei kann das Schlagteil als in einem Gehäuseteil der Vorrichtung geführter Kolben mit einem die Stossimpulse abgebenden Kopfteil und einem sich anschliessenden zylindrischen Hohlteil ausgebildet sein. In dem Hohlteil ist die die Federkräfte erzeugende zylindrische Druckfeder zumindest teilweise geführt, wobei es dabei zweckmässig ist, dass sich die Druckfeder mit ihrem einen Ende an der Rückseite des Kopfteils des Kolbens und mit ihrem andern Ende an einem Gehäuseteil der Vorrichtung abstützt.

In einem weiteren bevorzugten Ausführungsbeispiel soll die Vorrichtung zur Abgabe eines Stossimpulses auf das übertragende Element pneumatisch erfolgen. Dazu umfasst die Vorrichtung bevorzugter Weise ein Projektil.

Bevorzugt umfasst die Vorrichtung zur Abgabe eines Stossimpulses auch eine Einrichtung zur Beaufschlagung des Projektils mit Druckluft. In einem besonders bevorzugten Ausführungsbeispiel umfasst diese einen Kolben, der in einem Zylinder geführt ist. Dadurch kann vorteilhafter Weise, wenn der Kolben einen grösseren Querschnitt als das das Projektil aufweist, ein noch grössere Druck, je nach gewähltem Querschnittsflächenverhältnis, auf das Projektil aufgebracht werden.

Der Zylinder ist vorteilhafter Weise über einen Schlauch mit einem Kanal, in dem sich das Projektil befindet, verbunden. In diesem Kanal kann das Projektil beschleunigen, bis es auf das übertragende Element trifft.

Der Kanal zur Führung des Projektils kann in einem Lauf mit einem Gehäuse vorgesehen sein. An das Gehäuse können Stellflächen, Füße oder eine Aufhängevorrichtung angeformt sein.

Die Beschleunigung des Kolbens erfolgt bevorzugt mittels Federkraft. Dazu ist dem Kolben ebenfalls ein Federglied zugeordnet. Als Führung für das Federglied weist der Kolben vorteilhafter Weise einen Steg auf.

Zum Spannen und Freigeben der in dem Schlagteil bzw. der in dem Steg des Kolbens zur Beaufschlagung des Projektils mit Druckluft geführten Feder ist es vorteilhaft, wenn der Kolben des Schlagteils längs seines Aussenmantels mindestens eine Zahnstange aufweist, in die ein Ritzel eingreift, das nur auf seinem Teilumfang einen Zahnkranz besitzt, z.B. auf einem Teilumfang von 180°, wobei statt dem Ritzel auch eine Schnecke möglich ist. Statt einer separaten Zahnstange kann in den Aussenmantel des Schlagteils auch direkt eine Zahnung eingeformt sein.

Wird nun das Ritzel gedreht, dann bewegt sich das Schlagteil von der Sonde weg sobald der partielle Zahnkranz mit der Zahnstange im Eingriff steht und die in dessen Kolben geführte Feder wird dabei zusammengedrückt. Sobald nun der Zahnkranz des Ritzels die Zahnstange verlässt, also ausser Eingriff kommt, kann sich die komprimierte Feder schlagartig entspannen und deren freiwerdende Federkraft beschleunigt ein Projektil auf die gewünschte Geschwindigkeit. Dieses Projektil trifft auf die Sonde, erzeugt eine Stosswelle im Applikator der seinerseits eine entsprechende Stosswelle an das zu behandelnde Objekt weitergibt.

Die jeweiligen Drehungen des Ritzels erfolgen zweckmässigerweise motorisch, und hier mittels eines batteriegespeisten Elektromotors, dem man vorzugsweise ein Untersetzungsgetriebe vorschaltet, um auf brauchbare Drehzahlen des Ritzels zu kommen, welche die jeweiligen Schlagfrequenzen beeinflussen.

Im Rahmen der Erfindung soll aber nicht nur die eben beschriebene Anordnung liegen, sondern jede denkbare Haltevorrichtung, welche die gespannte Druckfeder hält und sie dann getriggert freigibt.

Bei einer Verwendung der erfindungsgemässen Vorrichtung für die intrakorporale Stosswellen-Lithotripie ist es zweckmässig, dass hier das die Stosswellen übertragende Element derart sondenförmig ausgeführt ist, damit sich dieses in ein Endoskop einführen lässt und zwar zum Zertrümmern von Konkrementen wie Nieren- oder Gallensteinen in einem lebenden Körper.

Dagegen bei einer Verwendung der erfindungsgemässen Vorrichtung für die extrakorporale Stosswellen-Orthopädie, Dermatologie und Ästhetik (Cellolite) ist es zweckmässig, dass deren die Stosswellen übertragendes Element als ein abgerundetes Kugelsegment zur Auflage auf den lebenden Körper ausgebildet ist. Diese Vorrichtung eignet sich zwar auch zur Zertrümmerung von Konkrementen von ausserhalb, jedoch können damit vor allem erkrankte Körperpartien eines Menschen wie bei Tieren, insbesondere in Bereichen der Sehnen, Bänder, Knochen sowie Haut (Zellulitis, Wunden) mit Stosswellen behandelt werden.

Zur guten Handhabung der erfindungsgemässen Vorrichtung ist es zweckmässig, dass diese als medizinisches Handgerät ausgebildet ist, d.h. dieses Handgerät sollte von seinen äusseren Abmessungen her gesehen lediglich eine Grösse aufweisen, die es erlaubt, dass das Gerät gut mit einer erwachsenen Hand festgehalten werden kann.

Diese als ein medizinisches Handgerät ausgebildete Vorrichtung kann neben einem Gehäuse für die Aufnahme des Stosswellengenerators auch vorteilhaft derart beschaffen sein, dass an dessen Gehäuse wahlweise ein Vorsatz mit einer Sonde für die Lithotripie oder ein solcher für die Orthopädie lösbar anbringbar ist. Mit einem derartigen Baukastensystem wird vorteilhaft die Typenvielfalt reduziert, was die Wirtschaftlichkeit eines derartigen Handgeräts merklich erhöht.

### Figurenbeschreibung

Weiter Merkmale und Vorteile der vorliegenden Erfindung ergeben sich aus der nachfolgenden Beschreibung bevorzugter Ausführungsbeispiele in Verbindung mit der Zeichnung; darin zeigen
Fig. 1 das Prinzip eines erfindungsgemässen Stosswellengenerators,
Fig. 2 eine perspektivische Ansicht eines medizinischen Handgerätes, das mit dem Stosswellengenerator gemäss Fig. 1 ausgestattet und für eine intrakorporalen Stosswellen-Lithotripie geeignet ist,
Fig. 3 eine perspektivische Ansicht eines bei dem Gerät nach Fig. 2 möglichen austauschbaren Vorsatz für die Verwendung bei einer extrakorporalen Stosswellen-Orthopädie in einem vergrösserten Massstab,
Fig. 4 bis 6 das Prinzip eines weiteren Ausführungsbeispiels des erfindungsgemässen Stosswellengenerators.

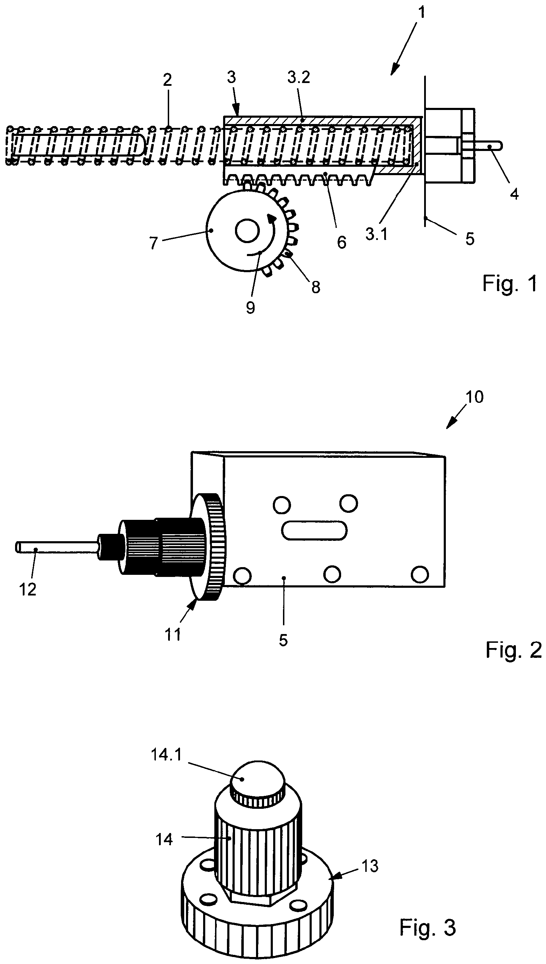

Das in Fig. 1 schematisch dargestellte Prinzip eines Stosswellengenerators 1 besitzt zur Erzeugung von Stossimpulsen ein als zylindrische Druckfeder ausgebildetes Federglied 2, das mit einem Schlagteil 3 in Wirkverbindung steht, welches wiederum die erzeugten Stossimpulse an ein Stosswellen übertragendes Element 4 weiter gibt. Die Druckfeder 2 besteht aus Federstahl, jedoch können auch andere Werkstoffe, wie z.B. Kunststoff, zum Einsatz kommen.

Das Schlagteil 3, welches in geeigneter Weise in einem Gehäuses 5 der in der Fig. 2 dargestellten Vorrichtung geführt ist, besteht aus einem Art Kolben mit einem die Stossimpulse abgebenden Kopfteil 3.1 und einem sich daran anschliessenden zylindrischen Hohlteil 3.2.

In dem Hohlteil 3.2 ist die Druckfeder 2 teilweise geführt und stützt sich mit ihrem rechten Ende an der Rückseite des Kopfteils 3.1 und mit ihrem linken Ende an einem nicht näher dargestellten Teilabschnitt des Gehäuses 5 ab. Das Schlagteil 3 selbst ist zu dem die Stosswellen übertragenden Element 4 koaxial angeordnet.

Ferner besitzt das Hohlteil 3.2 des Schlagteils 3 längs seines Aussenmantels eine integrale Zahnung oder angebrachte Zahnstange 6, in die ein Ritzel 7 oder eine Schnecke (nicht dargestellt) periodisch eingeklinkt werden kann. Das Ritzel 7 hat dazu nur auf einem Teil seines Umfangs einen Zahnkranz 8, dessen Profil dem Profil der Zahnstange 6 entspricht. Bei dem vorliegenden Ausführungsbeispiel ist der partielle Zahnkranz 8 auf einen Umfangsbereich von 180° begrenzt; dies wird bevorzugt, er kann jedoch auch grösser oder kleiner sein.

Das Ritzel 7 selbst wird über einen im Gehäuse 5 sich befindenden batteriegespeisten Elektromotor - angedeutet durch den Pfeil 9 in Fig. 1 - angetrieben, wobei diesem ein geeignetes Untersetzungsgetriebe vorgeschaltet ist.

Das in Fig. 2 vereinfacht dargestellte medizinische Handgerät - bezeichnet mit 10 - ist im vorliegenden Fall für die Verwendung bei der intrakorporalen Stosswellen-Lithotripie ausgelegt. Hierzu ist ein lösbarer Vorsatz 11 vorgesehen, dessen Stosswellen übertragendes Element - bezeichnet mit 12 - sondenförmig ausgeführt ist. Damit kann dieses Element 12 in ein Endoskop eingeführt werden und somit steht das distale Ende der Sonde für eine Zertrümmerung von Körpersteinen durch über die Sonde übertragene Stosswellen zur Verfügung.

Für den Fall, dass das Handgerät 10 gemäss Fig. 2 für ein extrakorporale Stosswellen-Orthopedie verwendet werden soll, kann der Vorsatz 11 auch gegen einen Vorsatz 13 (Fig. 3) ausgetauscht werden, dessen Stosswellen übertragendes Element - bezeichnet mit 14 - ein Art abgerundetes Kugelsegment 14.1 an seinem Kontaktbereich besitzt, das zur äusseren Auflage auf eine Körperpartie eines Menschen oder eines Tieres geeignet ist.

Beide Vorsätze 11 und 13 eignen sich für eine Zertrümmerung von Konkrementen wie Nieren-, Harn- oder Gallensteinen, wobei insbesondere der Vorsatz 13 besser für die Beaufschlagung mit Stosswellen auf erkrankte Körperbereich wie Sehnen- und Bänderbereiche, Knochenpartien, Hautstellen sowie schlechthin Wunden geeignet ist.

Das medizinische Handgerät 10 hat eine Grösse, die es erlaubt, dass es von einem erwachsenen Benutzer bequem in einer Hand gehalten werden kann.

Die Funktionsweise der vorliegenden Erfindung ist folgende:
Der Stosswellengenerator 1, der in dem Gehäuse 5 untergebracht ist, wird durch einen Elektromotor über ein Untersetzungsgetriebe (symbolisch Pfeil 9) entgegen dem Uhrzeigersinn mit ungefähr 5 Hz (300 Upm) angetrieben, wodurch der Zahnkranz 8 periodisch in die Zahnstange 6 eingreift und das Schlagteil 3 um die Länge der Zahnstange 6 nach links verschiebt und dabei die Druckfeder 2 um diesen Betrag zusammendrückt. Die dabei in der Druckfeder 2 sich speichernde Schlagenergie wird dann plötzlich, d.h. schlagartig frei, und zwar sobald der letzte Zahn des Zahnkranzes 8 die Zahnstange 6 verlässt. Mit dieser durch die kurzzeitige Entspannung der Druckfeder 2 zur Verfügung stehenden Federkraft wird sodann das Element 4 bzw. 12 bzw. 14 schlagartig beaufschlagt, wobei zwischen den Endabschnitten dieser Elemente und dem Kopfteil 3.1 quasi als Puffer noch ein Masseteil vorgesehen sein kann.

Durch den schlagartigen Aufprall des Kopfteils 3.1 auf das jeweilige Element 4 bzw. 12 bzw. 14 entstehen in diesem Stosswellen, die dann entweder zur Zertrümmerung von Körpersteinen oder aber z.B. zum Abbau von Kalkablagerungen in bestimmten Körperbereichen, wie in Bereichen der Schulter, verwendet werden. Wie oft nun dieser Aufprall pro sec. erfolgen soll, hängt wiederum von der Drehzahl des Ritzels 7 ab und diese wiederum von der gewünschten Intensität der jeweiligen Behandlung.

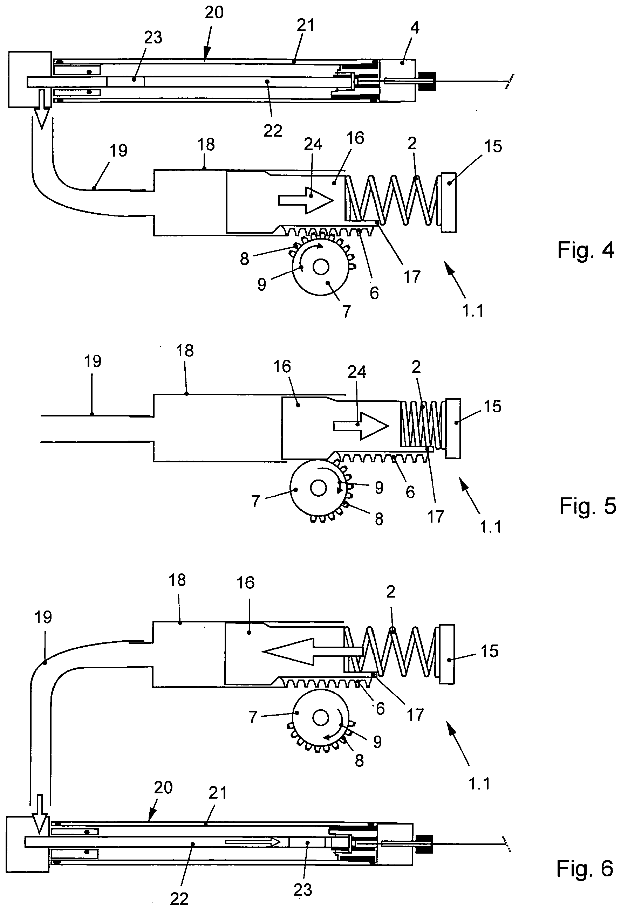

Die Figuren 4 bis 6 zeigen ein weiteres Ausführungsbeispiel eines erfindungsgemässen Stosswellengenerators 1.1. Dieser weist analog zum vorhergehenden Ausführungsbeispiel ein Federglied 2 auf. Dieses ist zwischen einer Anschlagplatte 15 und einem Kolben 16 eingebracht. Zum Führen des Federglieds 2 ist an den Kolben 16 ein Steg 17 angeformt. Der Kolben 16 wird in einem Zylinder 18 geführt. An diesen schliesst ein Schlauch 19 an, in den ggffs. zur besseren Steuerung bzw. Absperrung ein Ventil integriert sein kann. Der Schlauch 19 ist mit einem Lauf 20 verbunden. Der Lauf 20 umfasst ein Gehäuse 21 und ein Führungsrohr 22 für ein Projektil 23. An einem Ende des Laufs 20 ist ein übertragendes Element 4 angeordnet.

Der Kolben 16 und der Steg 17 weisen analog zum vorhergehenden Ausführungsbeispiel eine Zahnung 6 auf. Diese steht mit einem Ritzel 7, das nur teilweise einen Zahnkranz 8 aufweist, in Wirkverbindung.

Die Funktionsweise der vorliegenden Erfindung ist folgende:
Analog zum vorhergehend beschriebenen Ausführungsbeispiel wird auch der Stosswellengenerator 1.1 durch einen nicht dargestellten Elektromotor über ein Untersetzungsgetriebe, der Antrieb wird durch den Pfeil 9 symbolisiert, um den Uhrzeigersinn angetrieben. Dadurch greift der Zahnkranz 8 des Ritzels 7 in die Zahnung 6 ein, bewegt den Kolben 16 in Pfeilrichtung 24 und drückt das Federglied 2 gegen die Anschlagplatte 15. Dabei speichert das Federelement 2 Bewegungs- oder Schlagenergie. Diese wird dann plötzlich, d.h., schlagartig frei, wenn der letzte Zahn des Zahnkranzes 8 des Ritzels 7 die Zahnung 6 verlässt. Mit dieser durch die kurzzeitige Entspannung der Druckfeder 2 zur Verfügung stehenden Federkraft wird dann der Kolben 16 schlagartig, wie in Figur 6 dargestellt, entgegen der Pfeilrichtung 24 bewegt und drückt die sich im Zylinder 18 und Schlauch 19 befindliche Luft in den Kanal 22 des Laufs 20. Dadurch wird das Projektil 23 auf das übertragende Element 4 geschossen. Durch den schlagartigen Aufprall des Projektils 23 auf das übertragende Element 4 entstehen in diesem, analog zum vorhergehenden Ausführungsbeispiel, Stosswellen, die dann zu den bereits beschriebenen Anwendungen dienen können.

Insgesamt ist mit der vorliegenden Erfindung ein baukastenartiges wirtschaftliches medizinisches Handgerät geschaffen worden, das durch seine mechanische Arbeitsweise sehr robust und somit auch wartungsarm ist und eine relativ hohe exakte Schlagenergie und somit Stosswellen anbieten kann, was sowohl für den Benutzer als auch für den jeweiligen Patienten von grossem Vorteil ist.

**Bezugszeichenliste**

| | | | | | |
|---|---|---|---|---|---|
| 1 | Stosswellengenerator | 34 | | 67 | |
| 2 | Federglied; Druckfeder | 35 | | 68 | |
| 3 | Schlagteil | 36 | | 69 | |
| 3.1 | Kopfteil | | | | |
| 3.2 | Hohlteil | | | | |
| 4 | Element | 37 | | 70 | |
| 5 | Gehäuse | 38 | | 71 | |
| 6 | Zahnstange | 39 | | 72 | |
| 7 | Ritzel | 40 | | 73 | |
| 8 | Zahnkranz | 41 | | 74 | |
| 9 | Antrieb (E-Motor) | 42 | | 75 | |
| 10 | Handgerät | 43 | | 76 | |
| 11 | Vorsatz | 44 | | 77 | |
| 12 | Element | 45 | | 78 | |
| 13 | Vorsatz | 46 | | 79 | |
| 14 | Element | 47 | | | |
| 14.1 | Kugelsegment | | | | |
| 15 | Anschlagplatte | 48 | | | |
| 16 | Kolben | 49 | | | |
| 17 | Steg | 50 | | | |
| 18 | Zylinder | 51 | | | |
| 19 | Schlauch | 52 | | | |
| 20 | Lauf | 53 | | | |
| 21 | Gehäuse | 54 | | | |
| 22 | Kanal | 55 | | | |
| 23 | Projektil | 56 | | | |
| 24 | Pfeilrichtung | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Vorrichtung zum Einleiten von Stosswellen in einen lebenden Körper zur Zertrümmerung von Konkrementen wie Nieren-, Harn- oder Gallensteinen, wobei die Stosswellen durch mindestens ein die Stosswellen übertragendes Element (4; 12; 14) eingeleitet werden, auf das Stossimpulse einwirken, wobei zur Erzeugung der Stossimpulse mindestens ein Federglied (2) dient, dem eine Spanneinrichtung zugeordnet ist, **dadurch gekennzeichnet, dass** eine sich in dem Federglied (2) speichernden Schlagenergie schlagartig frei und auf ein Schlagteil (3, 16) übertragbar ist, über welches das Element (4, 12, 14) direkt oder indirekt über ein Projektil (23) schlagartig beaufschlagbar ist, wobei
das Schlagteil (3, 16) eine Zahnung oder Zahnstange (6) aufweist, in die ein Ritzel (7) eingreift, wobei das Ritzel (7) nur auf einem Teil seines Umfangs einen Zahnkranz (8) besitzt und deshalb geeignet ist, nur periodisch in die Zahnung oder Zahnstange (6) eingeklinkt zu werden.

2. Vorrichtung nach Anspruch 1, **dadurch gekennzeichnet, dass** als Federglied eine pneumatische oder zylindrische mechanische Druckfeder (2) dient, die mit einem Schlagteil (3) derart in Wirkverbindung steht, dass die Federkraft der Druckfeder (2) bei einer plötzlichen Freigabe ihre Druckspannung in der Form eines Stossimpulses auf das übertragende Element (4; 12; 14) übertragbar ist.

3. Vorrichtung nach Anspruch 2, **dadurch gekennzeichnet, dass** das Schlagteil (3) als in einem Gehäuseteil geführter Kolben mit einem die Stossimpulse abgebenden Kopfteil (3.1) und einem sich daran anschliessenden zylindrischen Hohlteil (3.2) ausgebildet ist.

4. Vorrichtung nach Anspruch 3, **dadurch gekennzeichnet, dass** in dem zylindrischen Hohlteil (3.2) die Druckfeder (2) zumindest teilweise geführt ist, wobei sich diese mit ihrem einen Ende an der Rückseite des Kopfteils (3.1) des Kolbens und mit ihrem anderen Ende an einem Gehäuseteil abstützt.

5. Vorrichtung nach Anspruch 4, **dadurch gekennzeichnet, dass** das Hohlteil (3.2) des Schlagteils (3) längs seines Aussenmantels mindestens eine Zahnung oder Zahnstange (6) aufweist, in die das Ritzel (7) oder die Schnecke eingreift.

6. Vorrichtung nach Anspruch 1, **dadurch gekennzeichnet, dass** der Stossimpuls auf das übertragende Element (4) durch ein Projektil (23) übertragbar ist.

7. Vorrichtung nach Anspruch 6, **dadurch gekennzeichnet, dass** das Projektil (23) mit Druckluft beaufschlagt ist.

8. Vorrichtung nach Anspruch 6, **dadurch gekennzeichnet, dass** eine Einrichtung zur Beaufschlagung des Projektils (23) mit Druckluft einen Kolben (16), der in einem Zylinder (18) geführt ist, aufweist.

9. Vorrichtung nach Anspruch 8, **dadurch gekennzeichnet, dass** der Zylinder (18) über einen Schlauch (19) mit einem Kanal (22), in dem sich das Projektil (23) befindet, verbunden ist, wobei ggfs. in dem Schlauch ein Ventil integriert ist.

10. Vorrichtung nach Anspruch 9, **dadurch gekennzeichnet, dass** der Kanal (22) zur Führung des Projektils (23) in einem Lauf (20) mit einem Gehäuse (21) vorgesehen ist.

11. Vorrichtung nach wenigstens einem der vorhergehenden Ansprüche, **dadurch gekennzeichnet, dass** der Kolben (16) als Führung für das Federglied (2) einen Steg (17) aufweist.

12. Vorrichtung nach wenigstens einem der vorhergehenden Ansprüche, **dadurch gekennzeichnet, dass** der Kolben (16) die Zahnung (6) aufweist.

13. Vorrichtung nach wenigstens einem der vorhergehenden Ansprüche, **dadurch gekennzeichnet, dass** der partielle Zahnkranz (8) auf einem Umfang des Ritzels (7) von 180° vorgesehen ist.

## Claims

1. Apparatus for introducing shock waves into a living body for disintegrating concretions such as kidney stones, urinary stones or gall stones, the shock waves being introduced by at least one element (4; 12; 14) which transmits the shock waves and on which shock pulses act, at least one spring member (2) serving to generate the shock pulses, the spring member (2) being assigned a tensioning device, **characterised in that** an impact energy being stored in the spring member (2) is abruptly released and can be transmitted to a percussion part (3, 16), via which the element (4, 12, 14) is abruptly acted on directly or indirectly via a projectile (23),
the percussion part (3, 16) having a toothing or rack (6), into which a pinion (7) engages, the pinion (7) having a toothed rim (8) only on a part of its circumference and therefore being suitable only for being engaged periodically with the toothing or rack (6).

2. Apparatus according to Claim 1, **characterised in that** a pneumatic or cylindrical mechanical compression spring (2) serves as the spring member and is operatively connected to a percussion part (3) such that the spring force of the compression spring (2), when its compressive stress is released suddenly, can be transmitted to the transmitting element (4; 12; 14) in the form of a shock pulse.

3. Apparatus according to Claim 2, **characterised in that** the percussion part (3) is formed as a piston which is guided in a housing part and has a head part (3.1) which emits the shock pulses and a cylindrical hollow part (3.2) which adjoins the head part (3.1).

4. Apparatus according to Claim 3, **characterised in that** the compression spring (2) is guided at least partially in the cylindrical hollow part (3.2), the compression spring (2) being supported at its one end on the rear side of the head part (3.1) of the piston and at its other end on a housing part.

5. Apparatus according to Claim 4, **characterised in that** the hollow part (3.2) of the percussion part (3) has along its outer casing at least a toothing or rack (6), into which the pinion (7) or the worm engages.

6. Apparatus according to Claim 1, **characterised in that** the shock pulse can be transmitted to the transmitting element (4) by a projectile (23).

7. Apparatus according to Claim 6, **characterised in that** the projectile (23) is subjected to the action of compressed air.

8. Apparatus according to Claim 6, **characterised in that** a device for subjecting the projectile (23) to the action of compressed air has a piston (16) which is guided in a cylinder (18).

9. Apparatus according to Claim 8, **characterised in that** the cylinder (18) is connected via a flexible tube (19) to a channel (22) in which the projectile (23) is located, a valve being integrated in the flexible tube, if appropriate.

10. Apparatus according to Claim 9, **characterised in that** the channel (22) for guiding the projectile (23) is provided in a barrel (20) having a housing (21).

11. Apparatus according to at least one of the preceding claims, **characterised in that** the piston (16) has a rib (17) as a guide for the spring member (2).

12. Apparatus according to at least one of the preceding claims, **characterised in that** the piston (16) has the toothing (6).

13. Apparatus according to at least one of the preceding claims, **characterised in that** the partial toothed rim (8) is provided over 180° of a circumference of the pinion (7).

## Revendications

1. Dispositif pour introduire des ondes de choc dans un corps vivant pour détruire des calculs tel que des calculs rénaux, des calculs urinaires ou des calculs biliaires, les ondes de choc étant introduites à travers au moins un élément de transmission d'ondes de choc (4; 12; 14) sur lequel agissent des impulsions de choc, pour la génération des impulsions de choc servant au moins un élément de ressort (2) auquel est associé un moyen de tension, **caractérisé par le fait qu'**une énergie d'impact s'accumulant dans l'élément de ressort (2) peut être transférée librement par impulsion sur un élément de frappe (3, 16) par l'intermédiaire duquel l'élément (4, 12, 14) peut être sollicité directement ou indirectement par l'intermédiaire d'un projectile (23),
dans lequel
l'élément de frappe (3, 16) présente une denture ou crémaillère (6) dans laquelle s'engage un pignon (7), le pignon (7) ne possédant une couronne dentée (8) que sur une partie de sa périphérie et convient, de ce fait, pour n'être encliqueté que périodiquement dans la denture ou crémaillère (6).

2. Dispositif selon la revendication 1, **caractérisé par le fait que** comme élément de ressort sert un ressort de compression mécanique ou pneumatique cylindrique (2) qui se trouve en communication active avec un élément de frappe (3) de sorte que la force de ressort du ressort de compression (2) puisse, lors d'une libération soudaine de sa contrainte de compression, être transmise librement sous forme d'une impulsion de choc sur l'élément de transmission (4; 12; 14).

3. Dispositif selon la revendication 2, **caractérisé par le fait que** l'élément de frappe (3) est réalisé sous forme de piston, guidé dans une partie de boîtier, avec une partie de tête (3.1) émettant des impulsions de choc et une partie creuse cylindrique (3.2) y raccordée.

4. Dispositif selon la revendication 3, **caractérisé par le fait que**, dans la partie creuse cylindrique (3.2), le ressort de compression (2) est au moins partiellement guidé, ce dernier s'appuyant par l'une de ses extrémités sur la face arrière de la partie de tête (3.1) du piston et par son autre extrémité sur une partie de boîtier.

5. Dispositif selon la revendication 4, **caractérisé par le fait que** la partie creuse (3.2) de l'élément de frappe (3) présente le long de son enveloppe extérieure au moins une denture ou crémaillère (6) dans laquelle s'engage le pignon (7) ou la vis sans fin.

6. Dispositif selon la revendication 1, **caractérisé par le fait que** l'impulsion de choc peut être transmise à l'élément de transmission (4) par un projectile (23).

7. Dispositif selon la revendication 6, **caractérisé par le fait que** le projectile (23) est sollicité par de l'air comprimé.

8. Dispositif selon la revendication 6, **caractérisé par le fait qu'**un dispositif destiné à solliciter le projectile (23) par de l'air comprimé présente un piston (16) qui est guidé dans un cylindre (18).

9. Dispositif selon la revendication 8, **caractérisé par le fait que** le cylindre (18) est relié par l'intermédiaire d'un tuyau flexible (19) à un canal (22) dans lequel se trouve le projectile (23), dans le tuyau flexible étant éventuellement intégrée une soupape.

10. Dispositif selon la revendication 9, **caractérisé par le fait que** le canal (22) est prévu, pour le guidage du projectile (23), dans un canon (20) avec un boîtier (21).

11. Dispositif selon au moins l'une des revendications précédentes, **caractérisé par le fait que** le piston (16) présente, comme guide de l'élément de ressort (2), une nervure (17).

12. Dispositif selon au moins l'une des revendications précédentes, **caractérisé par le fait que** le piston (16) présente la denture (6).

13. Dispositif selon au moins l'une des revendications précédentes, **caractérisé par le fait que** la couronne dentée partielle (8) est prévue sur une circonférence du pignon (7) de 180°.
